# EUROPEAN PATENT APPLICATION

(11) **EP 0 529 296 A2**
(43) Date of publication of application: **03.03.1993**
(21) Application number: 92112376.6
(22) Date of filing: 20.07.1992
(51) Int. Cl.: A61F 13/15, A61F 13/46

(54) **Unitized absorbent structure**

(30) Priority: 19.07.1991 US 732564
(71) Applicant: JOHNSON & JOHNSON INC., Montreal, Quebec H1V 2E4 (CA)
(72) Inventor: Chauvette, Gaetan, Lonevevil, QC J4K 2B9 (CA); Ramacieri, Patricia, Montreal H1E 3T4 (CA)
(74) Representative: Strehl Schübel-Hopf Groening & Partner

(57) **Abstract**

A unitized, composite, fluid absorbent structure for use in thin, disposable absorbent products such as sanitary napkins, diapers, incontinence pads, adult briefs, wound dressings and the like. The unitized, composite, fluid absorbent structure comprises a transfer layer (12) of hydrophilic fibers adhered to a reservoir layer (14) of compressed peat moss material having a higher density than the transfer layer. A diffuse interface is defined between the transfer and the reservoir layers forming a shared, three-dimensional boundary zone containing peat moss material interspersed with hydrophilic fibers. The invention also extends to a method for manufacturing the unitized, composite, fluid absorbent structure and to a method for improving the fluid absorption characteristics of a peat moss sheet.

## Description

### FIELD OF THE INVENTION

The invention relates to the art of manufacturing structures for absorbing body exudate. More specifically, the invention relates to a unitized, composite, fluid absorbent structure containing peat moss material which can be used in sanitary napkins, diapers, incontinence pads, adult briefs, wound dressings and the like. The unitized, composite, fluid absorbent structure is unusually absorbent and retentive, has a comparatively short fluid penetration time and has a relatively long shelf-life. The invention also extends to a method for manufacturing the unitized, composite, fluid absorbent structure and to a method for reducing the fluid penetration time of a peat moss sheet and also to considerably reduce the degradation of the fluid absorption characteristics of the peat moss sheet as a result of aging.

### BACKGROUND OF THE INVENTION

Traditionally, sanitary napkins for medium to high menstrual flow have been relatively thick and bulky containing an absorbent core made of fluffed hydrophilic materials such as wood pulp, rayon or cotton. These absorbent materials are attractive because they are fairly easy to manufacture at low cost while having, at least theoretically, a high absorbent capacity. However, this category of disposable absorbent products is known to possess severe deficiencies, failing in practice to provide the required protection against leakage and staining. More particularly, such fibrous materials currently employed suffer from poor fluid distribution properties and do not have the ability to disperse fluid within the entire available absorbent volume. As a result, absorbed fluid will tend to remain localized to a certain area of the absorbent core, causing saturation and collapse. This phenomenon often results in fluid overflow and leakage.

Another important disadvantage of sanitary napkins with thick absorbent cores resides in their inability to closely conform and follow the contour of the human body, achieving at best an intermittent contact surface between the fluid permeable side of the sanitary napkin and the integument of the wearer, which results in poor gasketing, increasing the likelihood of fluid leaking past the edges of the sanitary napkin and staining the wearer's clothes.

To address the above-discussed problems, a growing number of thinner and more flexible sanitary napkins have been developed, providing better fit, comfort and discretion, while being sufficiently absorbent to provide effective menstrual protection. The absorbent cores used in thin sanitary napkins include compressed sphagnum peat moss material which has a large proportion of extremely tiny pores and capillaries, providing the ability to absorb an appreciable quantity of fluid. The peat moss material swells as it absorbs fluid, however, the swelling does not cause a reduction of capacity for further absorbing fluid. Rather, the swelling contributes to the ability of the absorbent core to generally maintain the structural integrity of the absorbent structure in use. Peat moss material also has the unique capability of "drying" adjacent materials by continuing to pull or wick fluid away from them over a long time period such that virtually all the fluid is collected in and wicked throughout the peat moss core.

Although peat moss material has certain highly desirable fluid absorption properties, it is characterized by a relatively slow fluid penetration time. This drawback is particularly significant for applications where the rate of fluid release is high. Urinary incontinence is an example where the onrush of body fluid can be contained only by an absorbent structure which exhibits an ultra-short fluid penetration time.

In order to adapt a peat moss absorbent layer for such applications, it is common practice to provide a highly permeable, fibrous, fluid transfer layer on the peat moss layer, whose function is to quickly collect and then meter the fluid to the peat moss layer. Fluid discharged on such composite absorbent structure will instantaneously ingress the transfer layer due to its highly porous network. From the transfer layer, fluid migrates toward the high wicking peat moss layer by capillary action as a result of the substantial difference in wicking power therebetween. The fluid migration is well controlled, occurring at the rate of acceptance of the peat moss material. However, there are limits to what the transfer layer may achieve in assisting the peat moss layer to take up fluid. In certain situations, for example in urinary incontinence, when the onrush of fluid is intense, the controlled migration process between the transfer and peat moss layers is disturbed because the fluid mass released on the absorbent structure strikes through the transfer layer and starts to accumulate as a coherent body on the peat moss layer, potentially causing an overflow leakage.

Peat moss material also suffers from a loss of hydrophilicity due to aging. When freshly produced, a sheet of peat moss material is intensely hydrophilic. However, over time, the peat moss material gradually looses its affinity to absorb water, developing a hydrophobic barrier on its surface. As a result, presently available absorbent products using peat moss have a shorter than optimal shelf-life. If these products are not used within their intended shelf-life, they can become partially or totally ineffective as absorbents for their intended function.

An object of the present invention is to provide a fluid absorbent structure containing peat moss material, and a method for manufacturing same, for use in disposable absorbent products such as sanitary napkins, diapers, adult briefs, incontinence pads, wound dressings and the like, having an advantageously short fluid penetration time and the ability to retain superior fluid absorption properties over long time periods.

Another object of the invention is to provide a method for reducing the fluid penetration time of an absorbent layer of peat moss material and also to considerably reduce the degradation of the fluid absorption characteristics of the peat moss material as a result of aging.

### SUMMARY OF THE INVENTION

The foregoing objects of providing a thin fluid absorbent structure comprising a highly absorbent and wicking peat moss material with improved fluid penetration time and increased shelf-life has now been accomplished in accordance with the composition, products and methods of the present invention.

In accordance with the purposes of the invention, as embodied and fully described herein, the present invention features a unitized, composite, fluid absorbent structure comprising,
- a transfer layer of hydrophilic fibers, and
- a reservoir layer of peat moss material having a higher density than the transfer layer, the transfer and the reservoir layers being joined through a diffuse interface forming a shared, three-dimensional boundary zone containing peat moss material, interspersed with hydrophilic fibers, the boundary zone achieving a condition of intimate, fluid-communicative relationship between the transfer and the reservoir layers.

A highly desirable feature of this absorbent structure is the diffuse interface between the transfer and the reservoir layers, providing a three-dimensional zone where hydrophilic fibers interpenetrate the surface of the peat moss material, achieving a strong bond between the layers to prevent them from separating in use. In addition, the diffuse interface forms a gradual transition area between the different fluid absorbing media contributing to reduce the fluid penetration time of the reservoir layer by providing a fluid path of lesser resistance by comparison to a laminated absorbent structure with a sharp and abrupt transition between the layers.

In a preferred embodiment, the hydrophilic fibers and the peat moss material are united in the three-dimensional boundary zone by hydrogen bonding. The interwoven nature of the three-dimensional boundary zone provides a mechanical fiber interlocking action, enhancing the strength of the bond between the layers.

Advantageously, the absorbent structure of this invention is capable to maintain its excellent fluid absorption properties over long time periods. By using a fibrous material for the transfer layer which is capable of retaining a strong affinity for water over long time periods, such as cellulosic pulp for example, the formation or development of an undesirable, water-repelling barrier on the surface of the reservoir layer as a result of aging is inhibited because the surface of the reservoir layer is penetrated by hydrophilic fibers, opening-up its structure and inducing fluid to enter the peat moss material comprising the reservoir layer.

Hence, disposable absorbent products incorporating the principles of the present invention are unusually absorbent, have a comparatively short fluid penetration time for capturing a fluid discharge on contact and have an improved and relatively long shelf-life.

The invention also provides a method for enhancing the fluid absorption characteristics of a layer of peat moss material, the method comprising the steps of,
- applying on the layer of peat moss material a layer of hydrophilic fibrous material having a lower density than the density of the layer of peat moss material, and
- forming between the layers a three-dimensional boundary zone containing peat moss material interspersed with hydrophilic fibers, the boundary zone achieving a condition of intimate fluid-communicative relationship between the layers.

The invention also provides a method for manufacturing a unitized, composite, fluid absorbent structure, comprising the steps of,
- applying a layer of hydrophilic fibers having a water content of not less than about 7% based on the weight of the layer of hydrophilic fibers free of moisture on a layer of peat moss material having a water content not less than about 11% based on the weight of the layer of peat moss material free of moisture; and
- removing water at an interface between the layers to establish hydrogen bonds between the hydrophilic fibers and the peat moss material, uniting the layers in an intimate fluid-communicative relationship.

In a preferred embodiment, the hydrophilic fibrous layer and the peat moss layer are formed independently by wet laying. The layers are dewatered to achieve the desired water content levels, placed in superposition and co-calendered to produce a durable bond therebetween. Mechanically compressing the layers by a calendering operation has a dual purpose. Firstly, it expels water at the interface between the layers to create hydrogen bond links. Secondly, it causes the layers to interpenetrate each other, creating a three-dimensional adhesion zone.

Advantageously, the unitized, composite, fluid-absorbent structure may be subjected to mechanical tenderizing, such as perf-embossing or mircocorrugating, to enhance its flexibility and comfort potential. Intense mechanical working may be performed without risking delamination of the absorbent structure by virtue of the strong hydrogen bonds uniting the absorbent layers, assisted by the mechanical fiber interlocking action.

The invention also provides a disposable, fluid absorbent product, such as a sanitary napkin, a diaper, an incontinence pad, an adult brief, a wound dressing and the like, comprising:
- a fluid permeable cover sheet;
- a backing sheet of fluid impervious material; and
- a unitized, composite, fluid absorbent structure between said sheets, the fluid absorbent structure including:
   a) a transfer layer of hydrophilic fibers; and
   b) a reservoir layer of peat moss material, the reservoir layer having a higher density than the transfer layer, the layers being united in a face-to-face relationship through a diffuse interface defining a shared, three-dimensional boundary zone containing peat moss material interspersed with hydrophilic fibers and united to the hydrophilic fibers by hydrogen bonding, the boundary zone achieving a condition of intimate fluid-communicative relationship between the layers.

### BRIEF DESCRIPTION OF THE DRAWINGS

- Figure 1 is a perspective view of a unitized, composite, fluid absorbent structure, constructed in accordance with the present invention;
- Figure 2 is an idealized cross-sectional view of the absorbent structure illustrated in Figure 1, showing in greater detail the interrelation at the fiber level between the transfer and the reservoir layers of the absorbent structure;
- Figure 3 is a perspective view of co-calendering equipment used in manufacturing the unitized, composite, fluid absorbent structure according to the invention; and
- Figure 4 is a fragmentary perspective view of a sanitary napkin incorporating the unitized, composite, fluid absorbent structure according to the invention.

### DETAILED DESCRIPTION OF A PREFERRED EMBODIMENT

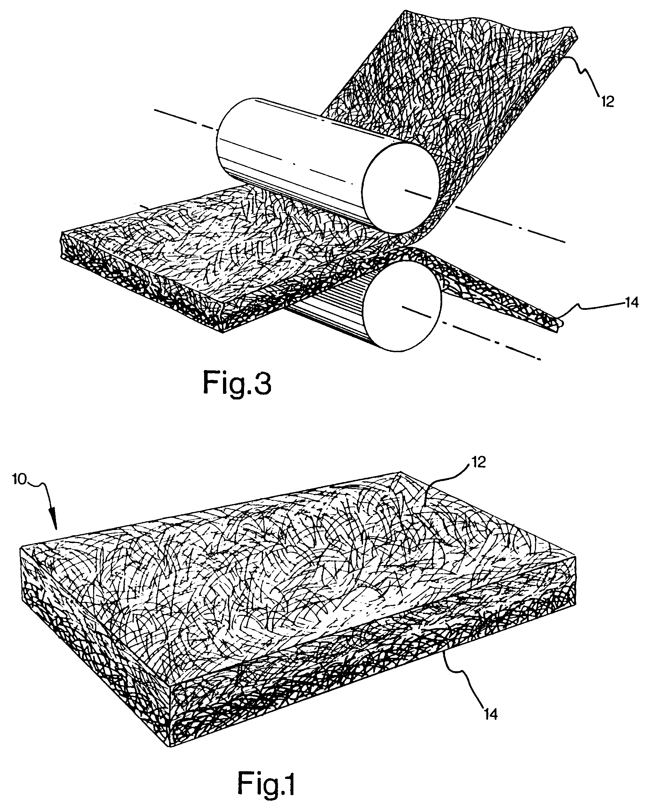

Referring to Figure 1, the reference numeral 10 designates comprehensively a unitized, composite, fluid absorbent structure embodying the principles of the present invention. Broadly stated, the absorbent structure 10 comprises a transfer layer 12 of hydrophilic fibrous material intimately associated with a denser reservoir layer 14 comprising peat moss material. The absorbent structure 10 has a large capacity, a high fluid absorption rate and it is capable of retaining its exceptional fluid absorption properties over long time periods.

The function of the transfer layer 12 is to receive the body fluid and hold the fluid until the denser reservoir layer 14 has an opportunity to absorb it. The transfer layer 12 is composed of cellulosic fibrous material such as sulfate, sulfite or Kraft wood pulp, debonded pulp, unbleached wood pulp or pulp bleached by chlorine processes or hydrogen peroxide, thermo-mechanical pulp or chemical thermal mechanical pulp.

The transfer layer 12 may be 100% cellulosic pulp or contain cellulosic pulp blended with polyester fibers, rayon fibers, cotton fibers or flexible foam (i.e. aminoether or low retention foam). Advantageously, the transfer layer 12 may be composed of a blend of cellulosic pulp with thermoplastic fibers for the purpose of stabilizing the transfer layer. For example, polyolefin fibers with the appropriate length and strength, such as low density polyethylene, or bicomponent fibers having polyethylene or polyester cores and a lower melting polyolefin sheath may be used, or polypropylene, polyvinylacetate, or other polyolefin fibers or thermoplastic pulp equivalents and the like. Blending such fibers with cellulosic pulp adds stability and integrity to the transfer layer material. The ratio of thermoplastic fiber to cellulosic pulp is preferably about 1:99 to about 50:50. More preferably, the ratio should be between about 3:97 and about 20:80. The fibers of the transfer layer may range in length from about 0.3 mm (0.0117 inch) for ground wood pulp to about 76.2 mm (3 inches) for the stabilizing thermoplastic fibers. Preferably, the fibers are between about 6.35 mm (0.25 inch) to about 25.4 mm (1 inch) in length if the transfer layer 12 is intended to be stabilized by thermal bonding at the fibers' points of contact.

The fibrous material of the transfer layer 12 is hydrophilic to rapidly absorb water. If the hydrophilicity of the fibers is not sufficient, it is possible to treat the fibrous material with a surfactant to increase its wettability.

Preferably, the basis weight of the transfer layer 12 is in the range from about 15 grams per meter squared (g/m²) to about 500 g/m². More preferably, the basis weight of the transfer layer is in the range from about 100 g/m² to about 225 g/m². Most preferably, the basis weight is in the range from about 125 g/m² to about 175 g/m².

The density of the transfer layer 12 should range from about 0.05 grams per cubic centimer (g/cc) to about 0.45 g/cc at 344.738 Pa (0.05 pounds per square inch (psi)) pressure. More preferably, the density should be in the range from about 0.10 g/cc to about 0.25 g/cc at 344.738 Pa (0.05 psi) pressure. Most preferably, the density should range from about 0.12 g/cc to about 0.15 g/cc at 344.738 Pa (0.05 psi) pressure.

Preferably, the thickness of transfer layer 12 is in the range from about 7.62 mm (0.03 inches (in)) to about 19.05 mm (0.75 in) at 344.738 Pa (0.05 psi) pressure. More preferably, the thickness is in the range from about 1.27 mm (0.05 in) to about 12.7 mm (0.50 in) at 344.738 Pa (0.05 psi) pressure. Most preferably, the thickness is in the range from about 20.32 mm (0.08 in) to about 6.35mm (0.25in) at 344.738Pa (0.05psi) pressure.

Immediately adjacent to and bonded to the transfer layer 12 is the reservoir layer 14. The reservoir layer 14 is a highly dense, fluid absorbent layer having a fine porosity. It has a large fluid holding capacity and it is extremely retentive. In essence, it acts as a capillary "pump" to pull fluid away from the transfer layer 12.

The reservoir layer 14 is made from a compressed sphagnum peat moss material in accordance with processes broadly described in U.S. patent number 4,473,440, K. Ovans, issued on September 25, 1984, and patents referred to therein. The reservoir layer 14 may also be formed by any one of the methods set forth in U.S. patents number 4,170,515, J-M Lalancette et al., issued on October 9, 1979; number 4,226,232, Y. Levesque, issued on October 7, 1990; number 4,215,692, Y. Levesque, issued on August 5, 1980; and number 4,507,122, Y. Levesque, issued on May 26, 1985.

The reservoir layer may have a fibrous component admixed therewith, as set forth in U.S. patent number 4,473,440. The fibrous component is suitably a natural or a synthetic textile fiber such as rayon, polyester, nylon, acrylic or the like, having a length of from about 6.35 mm (0.25) to 38.1 mm (1.5 in), most preferably about 12.7 mm (0.5 in) and a denier of from about 0.11 to 0.55 tex (1.0 to 5). The fibrous component may be present in an amount from about 2 to 20% by weight, most preferably from 4 to 8%. The peat moss material may also comprise other components such as wood pulp, synthetic wood pulp, thermo-mechanical pulp, chemical thermal mechanical pulp, mechanically ground pulp, polymers, surfactants, superabsorbents and the like.

Preferably, the basis weight of the reservoir layer 12 is in the range from about 25 g/m² to about 700 g/m². More preferably, the basis weight of the reservoir layer is in the range from about 100 g/m² to about 300 g/m². Most preferably, the basis weight is in the range from about 125 g/m² to about 200 g/m².

The density of the reservoir layer is in the range from about 0.05 g/cc to about 0.5 g/cc at 344.738 Pa (0.05 psi) pressure. More preferably, the density is in the range from about 0.10 g/cc to about 0.35 g/cc at 344.738 Pa (0.05 psi) pressure. Most preferably, the density is in the range from about 0.20 g/cc to about 0.30 g/cc at 344.738 Pa (0.05 psi) pressure.

Preferably, the thickness of the reservoir layer is in the range from about 1.27 mm (0.05 in) to about 19.05 mm (0.75 in) at 344.738 Pa (0.05 psi) pressure. More preferably, the thickness is in the range from about 2.03 mm (0.08 in) to about 12.7 mm (0.5 in) at 344.738 Pa (0.05 psi) pressure. Most preferably, the thickness is in the range from about 3.05 mm (0.12 in) to about 6.35 mm (0.25 in) at 344.738 Pa (0.05 psi) pressure.

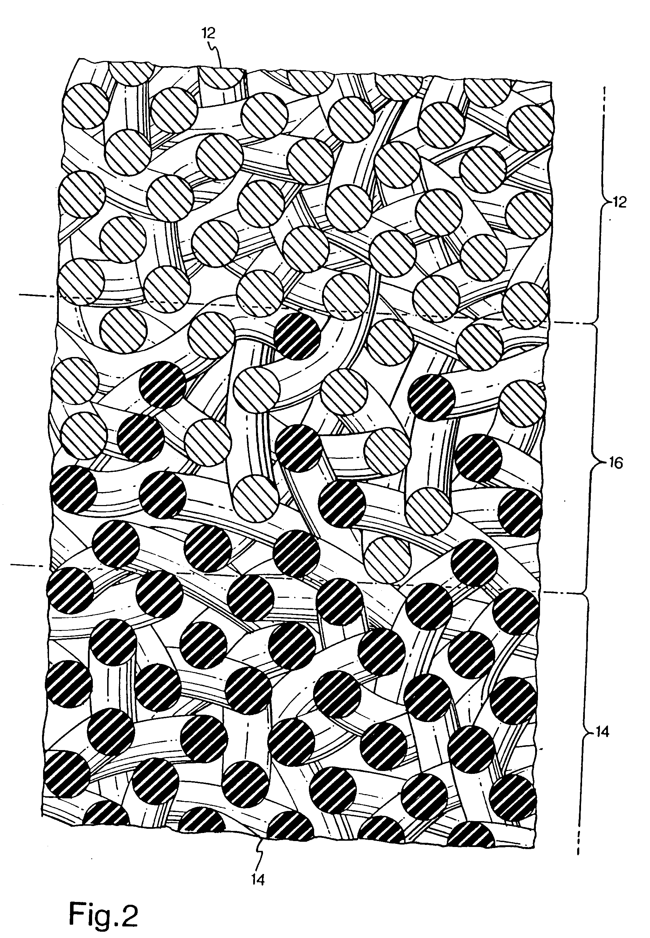

An important aspect of the invention resides in the intimate contact between the transfer layer 12 and the reservoir layer 14. This characteristic is best illustrated in Figure 2 which is an idealized cross-sectional view on a highly enlarged scale of the interface between the transfer layer 12 and the reservoir layer 14. To visually distinguish the various materials of the absorbent structure 10, the hydrophilic fibers of the transfer layer 12 are depicted as pale fibers while the peat moss material of the reservoir layer 14 is shown as dark fibers. It should be understood that this representation is solely for illustrative purposes and it does not necessarily conform to the true material structure of the layers 12 and 14.

The transfer layer 12 and the reservoir layer 14 meet along an interface 16 which is diffuse, forming a gradual transition from one layer to another. The interface 16 defines a three-dimensional boundary zone containing peat moss material interspersed with hydrophilic fibers. Notably, the concentration of peat moss material increases in the downward direction, providing a positive density gradient of peat moss material from the fluid transfer layer 12 toward the reservoir layer 14.

The hydrophilic fibers of the transfer layer 12 and the peat moss material of the reservoir layer 14 are united to one another at the interface 16 by hydrogen bonding, providing a strong and intimate adhesion. The bond strength is enhanced by the mechanical fiber interlocking action resulting from the interwoven nature of the interface 16. This combination of materials significantly improves the fluid penetration time into the reservoir layer 14 *per se*. Fluid, contained in the transfer layer 12, can more easily ingress the reservoir layer 14 because hydrophilic fibers penetrate the surface of the peat moss material, opening up its structure and providing a "lead-in" action by inducing fluid to enter the peat moss material by virtue of their affinity for water. This translates into a fluid path of lesser resistance toward the reservoir layer 14. In comparison, an absorbent structure with a sharp interface where the fibers of the fluid transfer layer do not penetrate within the reservoir layer provides a fluid path of higher resistance because fluid in egress from the transfer layer, suddenly encounters the much denser network of the reservoir layer, and is less able to penetrate therein without any assistance from pathways formed from penetrating fibers of the transfer layer.

Hence, the absorbent structure 10 is capable of a comparatively short fluid penetration time. In addition, the undesirable phenomenon of a hydrophobic barrier developing on the surface of the reservoir layer 14 as a result of aging of the peat moss material is less susceptible to occur, because hydrophilic fibers are implanted at the surface of the peat moss material, having the ability to draw fluid therein and open up the otherwise dense surface of the peat moss material.

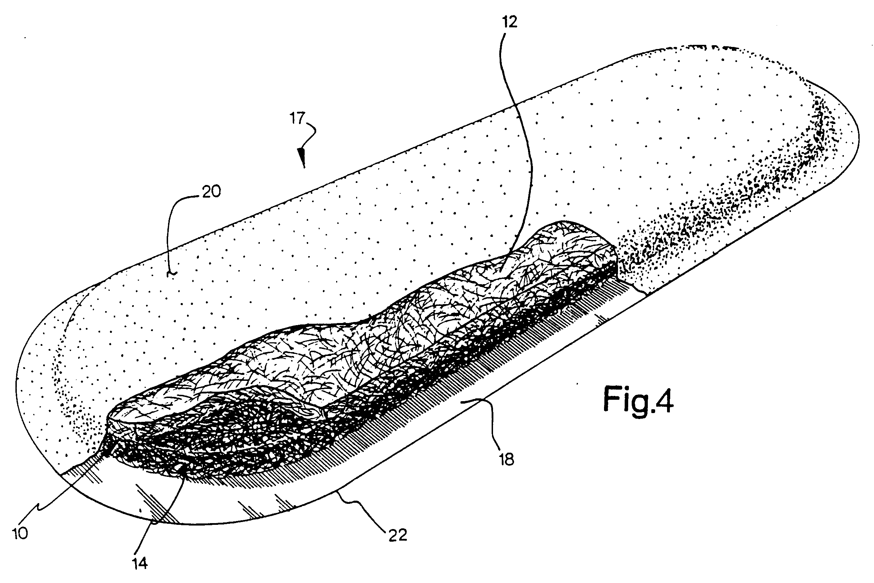

Figure 3 illustrates schematically the process for manufacturing the absorbent structure 10. The fluid transfer layer 12 and the reservoir layer 14 are independently formed as continuous webs by wet laying. The webs are subsequently dewatered to achieve the desired water content level in each web. The webs are then superposed and co-calendered by passing them between a pair of superposed rolls which exert a considerable pressure, expelling water from the webs and simultaneously causing the layers to interpenetrate each other, thus allowing hydrogen bonds to be created between the hydrophilic fibers and the peat moss material in the interpenetration zone and also mechanically interlocking the layers by virtue of fiber interengaging action. These hydrogen bonds, assisted by the mechanical interlocking action, intimately unite the webs together providing a unitized structure which can withstand intense mechanical working without delamination.

When cellulosic pulp is used for the transfer layer 12, the desired water content level of the web prior to co-calendering is in the range from about 7% to 11% based on the weight of cellulose in the web. For the peat moss material, a preferred water content level is in the range from about 11% to about 15% based on the weight of the peat moss web free of moisture.

Water content levels substantially under the lower limits of 7% and 11% for cellulosic pulp and peat moss material respectively, will adversely affect the formation of the hydrogen bonds. Increasing the water content levels beyond the upper limits of 11% and 15% for the cellulosic pulp and peat moss material respectively, is undesirable for practical reasons, mostly because the increased fluidity of the webs creates handling problems.

In order to tenderize, soften and improve the flexibility of the absorbent structure, it may be subjected to mechanical working such as perf-embossing or microcorrugating as described in the United States patents granted to Personal Products Company, numbers 4,596,567 and 4,559,050, issued on June 24, 1986 and December 17, 1985, respectively.

Broadly stated, the pert-embossing operation consists of perforating by shearing action, at a multiplicity of points the absorbent structure 10 to open up its structure by locally disrupting the integrity of the fibrous network. Subsequently, the absorbent structure is embossed transversely and longitudinally by passing the absorbent structure between rolls having intermeshing flutes. The created embossing lines constitute hinges considerably enhancing the flexibility of the absorbent structure.

The microcorrugating operation is similar to the perf-embossing except that no perforation is performed. The absorbent structure is solely subjected to the embossing operation to create closely spaced hinge lines.

Figure 4 illustrates a sanitary napkin incorporating the absorbent structure according to the invention. The sanitary napkin, designated comprehensively by the reference numeral 17, comprises an envelope 18 defining an internal space receiving the absorbent structure 10. The envelope 18 includes a fluid-permeable cover layer 20 made of a non-woven fabric or any other suitable porous web, and a fluid-impervious backing layer 22, made of polyethylene film for example. The cover and backing layers 20 and 22 are heat-sealed to one another along their marginal portions.

To attach the sanitary napkin 17 to the wearer's underpants, the fluid impervious backing layer 22 may be provided with adhesive zones covered with a pealable backing (not shown in the drawings).

Sanitary napkins constructed in accordance with the present invention are found to possess a very high fluid absorption capacity and a comparatively high fluid penetration rate which reduces the risk of failure when a large quantity of fluid is suddenly released on the sanitary napkin. In addition, the sanitary napkin is capable to retain its absorption properties over relatively long time periods, increasing the shelf-life of the product.

The scope of the present invention is not limited by the description, examples and suggestive uses herein, as modifications can be made without departing from the spirit of the invention. Applications of the product and methods of the present invention for sanitary and other health-care uses can be accomplished by any sanitary protection, incontinence, medical and absorbent methods and techniques as are presently or prospectively known to those skilled in the art. Thus, it is intended that the present application cover the modifications and variations of this invention provided that they come within the scope of the appended claims and their equivalents.

## Claims

1. A unitized, composite, fluid absorbent structure (10), comprising:
- a transfer layer (12) of substantially hydrophilic fibers; and
- a reservoir layer (14) of peat moss material, said reservoir layer having a higher density than said transfer layer (12), said layers (12, 14) being joined through a diffuse interface defining a shared, three-dimensional boundary zone containing peat moss material interspersed with hydrophilic fibers, said boundary zone achieving a condition of intimate fluid-communicative relationship between said layers.

2. A unitized, composite, fluid absorbent structure as defined in claim 1, wherein said hydrophilic fibers are united to said peat moss material in said three-dimensional boundary zone by hydrogen bonding.

3. A unitized, composite, fluid absorbent structure as defined in claim 1, wherein said hydrophilic fibers and said peat moss material are mechanically interlocked in said three-dimensional boundary zone.

4. A unitized, composite, fluid absorbent structure as defined in claim 1, wherein said absorbent structure is mechanically tenderized to increase its flexibility.

5. A unitized, composite, fluid absorbent structure as defined in claim 4, wherein said absorbent structure is mechanically tenderized by a process selected from the group consisting of perf-embossing and microcorrugating.

6. A unitized, composite, fluid absorbent structure as defined in claim 1, wherein said boundary zone has a positive density gradient of peat moss material in a direction from said transfer layer to said reservoir layer.

7. A unitized, composite, fluid absorbent structure, as defined in claim 1, wherein said transfer layer comprises fibers treated with a surfactant.

8. A unitized, composite, fluid absorbent structure as defined in claim 1, wherein said transfer layer includes material selected from the group consisting of sulfate, sulfite, debonded, bleached, unbleached, Kraft wood pulp, thermo-mechanical pulp, chemical thermal mechanical pulp, wood pulp bleached by a chlorine process and wood pulp bleached by hydrogen peroxide.

9. A unitized, composite, fluid absorbent structure as defined in claim 1, wherein said transfer layer includes a blend of cellulosic pulp and thermoplastic fibers.

10. A unitized, composite, fluid absorbent structure as defined in claim 1, wherein said transfer layer has a thickness in the range from about 0.762 mm (0.03 in) to about 19.05 mm (0.75 in) measured at 344.738 Pa (0.05 psi) pressure.
